# FASCICULE DE BREVET EUROPEEN

(11) **EP 4 255 512 B1**
(45) Date de publication et mention de la délivrance du brevet: **01.01.2025**
(21) Numéro de dépôt: 21816473.9
(22) Date de dépôt: 30.11.2021
(51) Int. Cl.: A61L 9/12, B60H 3/00

(54) **DIFFUSEUR SE FIXANT SUR UNE GRILLE D'AERATION POUR LA DIFFUSION CONTROLEE D'UN PARFUM D'AMBIANCE**
DIFFUSOR ZUR BEFESTIGUNG AN EINEM LÜFTUNGSGITTER ZUR GESTEUERTEN ABGABE EINES LUFTERFRISCHERS
DIFFUSER ATTACHING ON A VENTILATION GRILL FOR THE CONTROLLED DISPENSING OF AN AIR FRESHENER

(30) Priorité: 02.12.2020 FR 2012551
(43) Date de publication de la demande: 11.10.2023
(73) Titulaire: Produits Berger, 27520 Grand Bourgtheroulde (FR)
(72) Inventeur: OZOUF, Laurent, 76940 Notre-Dame-de-Bliquetuit (FR); GUILLARD, Christelle, 76130 Mont Saint Aignan (FR)
(74) Mandataire: Novagraaf Technologies
(86) Numéro de dépôt international: PCT/EP2021/083515
(87) Numéro de publication internationale: WO 2022/117545

(56) Documents cités:
- EP-A1- 1 721 767
- EP-B1- 1 721 767
- CN-U- 211 797 865
- FR-A1- 2 833 531
- FR-B1- 2 833 531

## Description

### Domaine technique de l'invention

La présente invention concerne un diffuseur de parfum, d'une composition parfumée ou d'une odeur.

L'invention concerne plus particulièrement un diffuseur prévu pour être fixé de manière amovible en vis-à-vis d'une grille d'un système d'aération de ventilation et/ou de climatisation, notamment d'un habitacle de véhicule automobile, de manière à être traversé axialement par un flux d'air à parfumer.

### Arrière-plan technique

On connaît la conception générale d'un tel diffuseur qui est fixé, par exemple par « clipsage » ou emboîtement élastique, sur une grille de ventilation grâce à une pince élastique.

Le boîtier est une coque qui peut être ouverte pour y mettre en place un bloc support d'un produit ou d'une composition parfumée, et par exemple un bloc en céramique parfumé.

Le flux d'air sortant à travers la grille de ventilation entraîne ainsi le parfum à travers le boîtier de manière à parfumer l'habitacle.

La conception générale d'un tel diffuseur est connue du document FR-A1 -27648806.

Il propose un diffuseur de parfum à plusieurs compartiments se fixant de manière amovible sur une grille d'aération, destiné à parfumer tous les lieux, aussi bien dans la voiture que dans la maison, ou au bureau, comportant un système d'aération ou de climatisation en diffusant régulièrement dans l'atmosphère un parfum composé de substances volatiles complexes par périodes successives. Le boîtier comporte à l'arrière une pince élastique rotative et interchangeable montée sur un socle circulaire permettant de l'orienter dans le sens désiré et permettant une utilisation sur tous types d'aérateurs, le boîtier contenant un matériau parfumé et étant aménagé en plusieurs compartiments isolés séparés par des cloisons étanches, chaque compartiment pouvant être mis en fonction successivement au moment désiré. Le fond de chaque compartiment est pourvu d'ouvertures par lesquelles s'effectue la diffusion du produit parfumé hors du boîtier, une partie au moins de ces compartiments ayant leur fond recouvert d'une étiquette auto-adhésive, munie d'un onglet d'arrachage, et obturant de façon étanche les ouvertures correspondantes, le retrait de chaque étiquette mettant en service un compartiment.

Un couvercle amovible est prévu pour fermer l'ensemble.

La conception connue d'un tel système de diffusion ne permet pas de moduler de manière contrôlée, voire de stopper la diffusion du parfum.

Le seul moyen pour l'utilisateur de moduler l'intensité du parfum à l'intérieur de l'habitacle est de faire varier la vitesse de la ventilation.

De plus, à l'arrêt du système de ventilation, l'évaporation continue à se produire de manière passive, par les ouvertures prévues à cet effet que comporte le boîtier.

Le document FR-A1-3026308 décrit et représente un dispositif de diffusion d'au moins un agent volatil comprenant une entrée d'air, un réservoir pour contenir une quantité déterminée d'agent volatil, une sortie d'air et un ventilateur adapté pour générer un flux d'air circulant depuis l'entrée d'air vers la sortie d'air en traversant le réservoir. Dans ce document, des volets pivotant d'obturation peuvent obturer sélectivement des ouvertures du boîtier du réservoir. Avec un flux d'air constant généré à l'aide du ventilateur, la cavité du réservoir peut être plus ou moins obturée, en générant ainsi un flux d'air plus ou moins important circulant à travers le dispositif

L'invention vise à proposer un diffuseur prévu pour être fixé de manière amovible en vis-à-vis d'une grille d'un système d'aération de ventilation et dont la conception permet de régler le flux d'air parfumé et pouvant par exemple permettre d'arrêter la diffusion du parfum.

L'état de la technique est aussi illustré par les documents EP1721767B1, CN211797865U et FR2833531A1.

### Résumé de l'invention

L'invention propose un diffuseur de parfum qui comporte :
- un élément arrière de fixation du diffuseur ;
- un boîtier avant, dans lequel deux parois axialement opposées arrière et avant du boîtier comportent chacune au moins une fenêtre, arrière d'entrée et avant de sortie respectivement, pour permettre la formation d'un flux d'air s'écoulant axialement à travers le boîtier et la diffusion d'un flux d'air parfumé hors du boîtier;
   - un support d'un matériau parfumé logé dans le boîtier avant ;
   - deux volets externes opposés, arrière et avant, d'obturation dont chacun est adjacent à une paroi associée, arrière et avant respectivement, du boîtier avant, qui sont accouplés pour former un ensemble de volets d'obturation qui est monté tournant par rapport au boîtier, et dont chacun comporte au moins une ouverture, arrière d'entrée et avant de sortie respectivement, pour permettre le passage du flux d'air à travers le boîtier lorsque ces ouvertures sont positionnées angulairement au moins partiellement en vis-à-vis des fenêtres arrière et avant associées du boîtier ; dans lequel l'ensemble de volets d'obturation est lié en rotation à l'élément arrière de fixation et le boîtier avant est monté tournant par rapport à l'élément arrière de fixation pour permettre un réglage de la position angulaire du boîtier avant par rapport à l'ensemble de volets d'obturation, et ainsi un réglage de la section de passage du flux d'air à travers le boîtier.

Selon d'autres caractéristiques de l'invention :
- l'élément arrière de fixation comporte une tige axiale de fixation qui est liée en rotation au volet arrière d'obturation et se prolonge vers l'avant à l'intérieur du boîtier avant ;
- le boîtier avant est un boîtier rechargeable comportant :
- un demi-boîtier arrière qui est monté tournant par rapport à l'arbre central,
- un demi-boîtier avant qui est porté par le demi-boîtier arrière sur lequel il est monté mobile entre une position fermée d'utilisation et une position ouverte permettant le rechargement du boîtier avant,
- dans lequel le volet avant d'obturation est monté libre en rotation par rapport au demi-boîtier avant, et le diffuseur comporte un dispositif de liaison en rotation du volet avant d'obturation ;
- l'arbre central lorsque le demi-boîtier avant est en position fermée ;
- le demi-boîtier avant est monté pivotant sur le demi-boîtier arrière, entre ses positions fermée d'utilisation et ouverte de rechargement, autour d'un axe de pivotement orthogonal à l'axe de rotation du boîtier avant ;
- le diffuseur comporte des moyens magnétiques, agencés entre les deux demi-boîtiers arrière et avant, qui maintiennent le demi-boîtier avant en position fermée d'utilisation ;
- le dispositif de liaison en rotation du volet avant d'obturation avec l'arbre central est un système magnétique comportant au moins un aimant ;
- le dispositif de liaison en rotation du volet avant d'obturation avec l'arbre central est un système magnétique de positionnement angulaire automatique, à la fermeture du boîtier avant du volet avant d'obturation par rapport à l'arbre central, et ainsi par rapport au volet arrière d'obturation ;
- le système magnétique de positionnement angulaire automatique, à la fermeture du boîtier avant, du volet avant d'obturation par rapport à l'arbre central comporte :
   - un élément magnétique arrière à aimantation permanente agencé à l'extrémité avant de l'arbre central qui comprend au moins deux pôles magnétiques opposés ;
   - un élément magnétique avant à aimantation permanente porté par le volet avant d'obturation et agencé en vis-à-vis du premier élément magnétique qui comprend deux pôles magnétiques opposés complémentaires des deux pôles magnétiques opposés du premier élément magnétique ;
   - le support d'un matériau parfumé comporte une face avant orthogonale à l'axe de l'arbre central, et ladite extrémité avant de l'arbre central s'étend sensiblement dans le plan de ladite face avant.

En variante :
- le demi-boîtier avant est emboîté élastiquement sur le demi-boîtier arrière ;
- le boîtier avant est partagé en deux demi-boîtiers arrière et avant selon un plan de joint orthogonal monté vissé sur le demi-boîtier arrière.

Selon différents exemples de réalisation :
- le boîtier avant est agencé axialement entre les deux volets externes opposés arrière et avant d'obturation ;
- ou bien les deux volets externes opposés arrière et avant d'obturation sont agencés axialement à l'intérieur du boîtier avant, chacun adjacent à la paroi associée, arrière et avant respectivement, du boîtier avant ;
- ou bien encore, un seulement des deux volets externes opposés arrière ou avant d'obturation est agencé axialement à l'intérieur du boîtier avant adjacent à la paroi associée, arrière ou avant respectivement, du boîtier avant.

### Brève descriptions des figures

D'autres caractéristiques et avantages de l'invention apparaîtront au cours de la lecture de la description détaillée qui va suivre pour la compréhension de laquelle on se reportera aux dessins annexés dans lesquels :

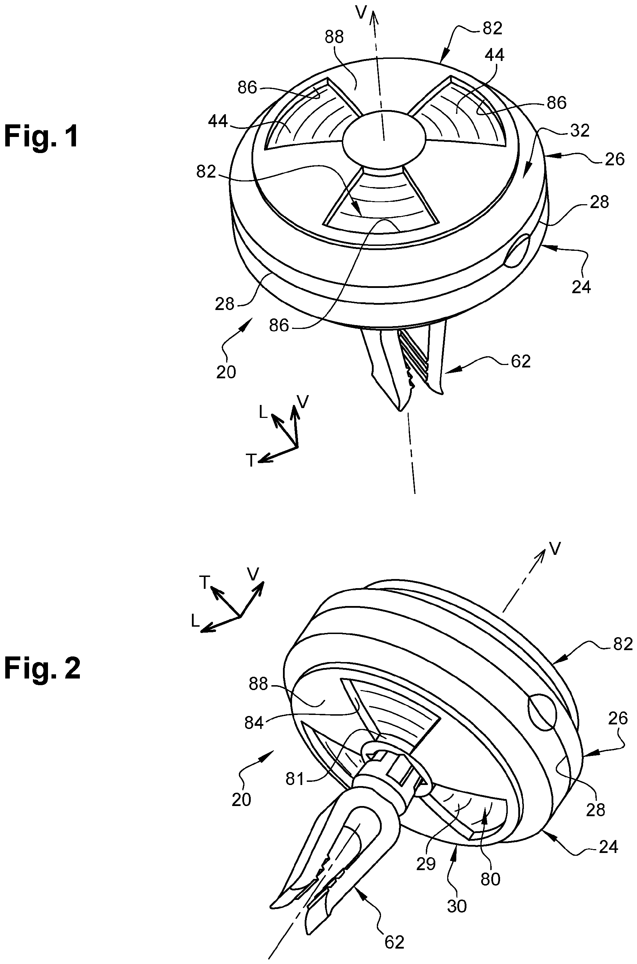

[Fig.1] - la figure 1 est une vue générale en perspective et de dessus d'un premier exemple de réalisation d'un diffuseur selon l'invention, à volets extérieurs, dont le boîtier est en position fermée d'utilisation et dont les volets couplés d'obturation sont en position fermée d'occultation complète des ouvertures du boîtier ;
[Fig.2] - la figure 2 est une vue en perspective de dessous du diffuseur de la figure 1 ;
[Fig.3] - la figure 3 est une vue en coupe par un plan axial médian du diffuseur de la figure 1 ;
[Fig.4] - la figure 4 est une vue en coupe par un autre plan axial du diffuseur de la figure 1 qui est représenté sans son demi-boîtier avant ;
[Fig.5] - la figure 5 est une vue analogue à celle de la figure 1 sur laquelle les volets couplés d'obturation sont en position ouverte dégageant complètement les ouvertures du boîtier ;
[Fig.6] - la figure 6 est une vue générale en perspective et de dessus du diffuseur selon l'invention dont le boîtier est en position ouverte de rechargement et dont les volets d'obturation sont en position partiellement ouverte d'occultation partielle des ouvertures du boîtier ;
[Fig.7] - la figure 7 est une vue analogue à celle de la figure 6 sur laquelle le diffuseur est représenté sans son support de matériau parfumé ;
[Fig.8] - la figure 8 est une vue analogue à celle de la figure 1 sur laquelle le diffuseur est représenté sans les composants constitutifs de son boîtier est sans le support de matériau parfumé ;
[Fig.9] - la figure 9 est une vue en perspective et en section par un plan vertical médian du boîtier dont les deux demi-boîtiers sont représentés en position fermée ;
[Fig.10] - la figure 10 est une vue en perspective et en section par un plan vertical médian du support de matériau parfumé ;
[Fig.11] - la figure 11 est une vue analogue à celle de la figure 1 qui représente un deuxième exemple de réalisation d'un diffuseur selon l'invention, à volets intérieurs ;
[Fig.12] - la figure 12 est une autre vue, analogue à celle de la figure 2, du diffuseur de la figure 11 ;
[Fig.13] - la figure 13 est une autre vue, analogue à celle de la figure 3, du diffuseur de la figure 11 ;
[Fig.14] - la figure 14 est une autre vue, analogue à celle de la figure 4, du diffuseur de la figure 11 représenté avec son demi-boîtier avant ;
[Fig.15] - la figure 15 est une autre vue, analogue à celle de la figure 8, du diffuseur de la figure 11 représenté en coupe par un plan axial ;
[Fig.16] - la figure 16 est une autre vue, analogue à celle de la figure 9, du boîtier avant du diffuseur de la figure 11.

### Description détaillée de l'invention

### Premier exemple de réalisation

Pour la description de l'invention et la compréhension des revendications, on adoptera à titre non limitatif et sans référence limitative à la gravité terrestre les orientations verticale, longitudinale et transversale selon le repère V, L, T indiqué aux figures dont les axes longitudinal L et transversal T s'étendent dans un plan horizontal.

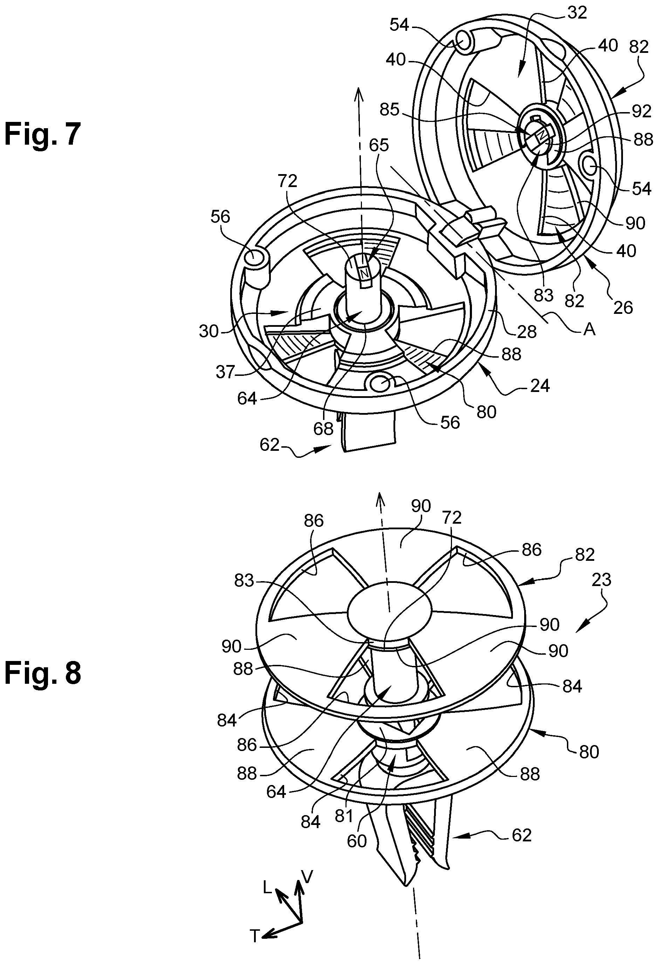

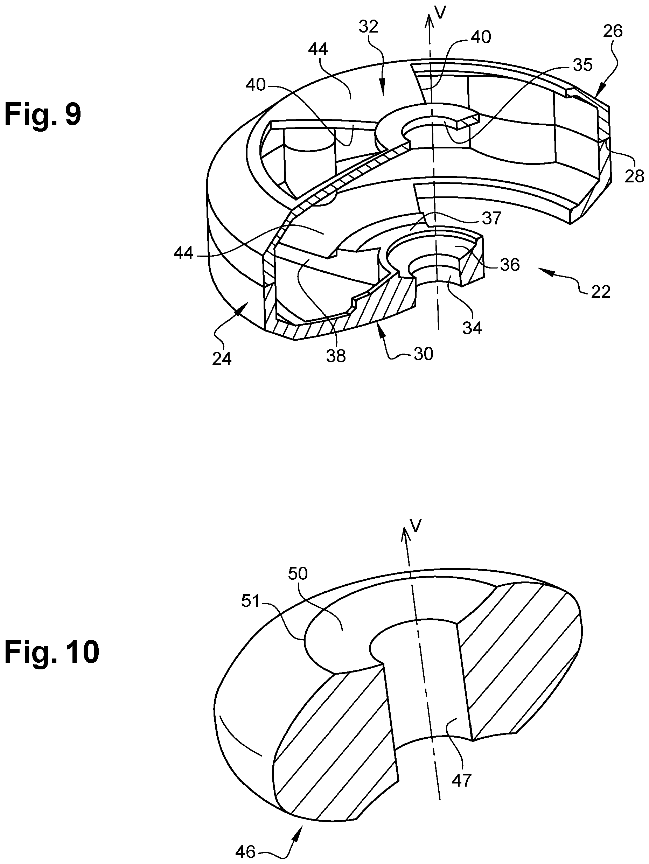

Par convention, l'axe vertical V est orienté de l'arrière vers l'avant et selon le sens de traversée du diffuseur par un flux d'air à parfumer. Dans la description qui va suivre, des éléments identiques, similaires ou analogues seront désignés par les mêmes chiffres de référence. On a représenté aux figures un diffuseur 20 qui est essentiellement constitué d'un boîtier avant 22 (Voir figure 9) qui est monté tournant autour de l'axe central V par rapport à un ensemble fixe 23 (Voir figure 8) comportant notamment des moyens arrière de fixation du diffuseur 20 et des moyens d'occultation du flux d'air traversant le boîtier avant 22.

Le boîtier avant 22 comporte un demi-boîtier arrière 24 et un demi-boîtier avant 26 qui sont complémentaires et qui sont raccordés selon un plan de joint 28 pour former une coque creuse de forme générale cylindrique qui est délimitée axialement par une paroi latérale arrière 30 appartenant au demi-boîtier arrière 24 et par une paroi latérale opposée avant 32 appartenant au demi-boîtier avant 26.

En son centre, chaque paroi latérale 30,32 comporte un trou 34,35 respectivement.

Dans le demi-boîtier arrière 24, le trou central 34 est entouré par un premier siège central annulaire tronconique concave 36 et par un deuxième siège annulaire périphérique convexe 37.

À titre d'exemple non limitatif, chaque paroi latérale arrière 30 et avant 32 comporte ici trois fenêtres, arrière d'entrée 38 et avant de sortie 40 respectivement, de forme générale en secteur triangulaire, qui sont réparties angulairement de manière régulière autour de l'axe V et qui sont séparées l'une de l'autre par trois portions pleines de forme générale en secteur triangulaire 42, 44.

Ainsi, chaque fenêtre 38, 40 s'étend ici sensiblement sur soixante degrés d'angle.

La conception du boîtier avant 22 est telle que - dans cet exemple et à titre non limitatif - en position fermée d'utilisation, les fenêtres 38,40 sont alignées angulairement.

La fonction du boîtier avant 22 est de contenir un support 46 d'une substance ou d'une composition parfumée dont la surface est en contact avec un flux d'air qui traverse le boîtier avant 22 en passant à travers les fenêtres 38 et 40.

Le support 46 est ici un galet en céramique imprégnée.

Le support 46 est en forme générale d'un tore qui est percé centralement par un alésage axial 47.

Le support 46 présent une symétrie générale de conception par rapport à un plan horizontal médian orthogonal à son axe central.

Dans chacune de ses deux faces opposées, le support 46 comporte une surface centrale annulaire concave 50 qui est complémentaire de celle du deuxième siège annulaire périphérique convexe 37 du demi-boîtier arrière 24 qui permet la mise en place et le centrage de support 46 dans le boîtier avant 22.

A titre de variante non représentée pour ce premier exemple de réalisation, le siège convexe complémentaire peut appartenir à la collerette 68 de l'arbre 64.

Dans ce cas, le support 46 est porté par l'arbre central 64 dont le diamètre est augmenté pour correspondre à celui de l'alésage central 47 du support 46. (Un exemple de cette variante est représenté plus avant dans le cadre du deuxième exemple de réalisation de l'invention).

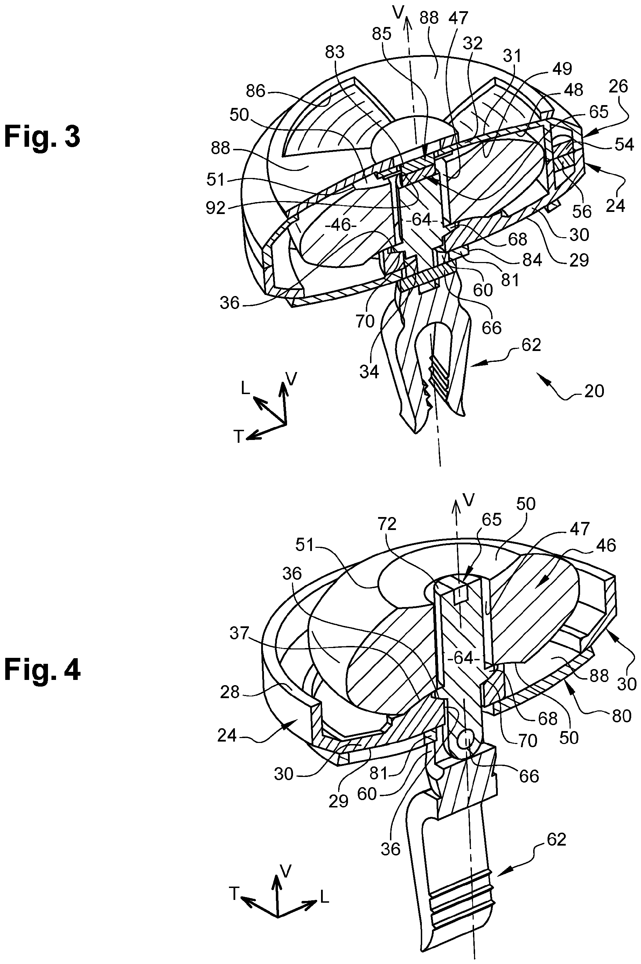

En position fermée du boîtier, et comme on peut le voir notamment à la figure 3, le support 46 est maintenu légèrement serré axialement entre le deuxième siège annulaire périphérique convexe 37 et une portion en vis-à-vis 49 de la face interne 48 de la paroi latérale avant 32 du demi-boîtier avant 26.

Le support 46 est aussi maintenu légèrement serré radialement entre les logements des aimants de fermeture.

Dans l'exemple illustré aux figures et à titre non limitatif, le demi-boîtier avant 26 est monté articulé par rapport au demi-boîtier arrière 24 au moyen d'une charnière 52.

Le demi-boîtier avant 26 est ainsi monté pivotant autour d'un axe horizontal A qui est orthogonal à l'axe central V.

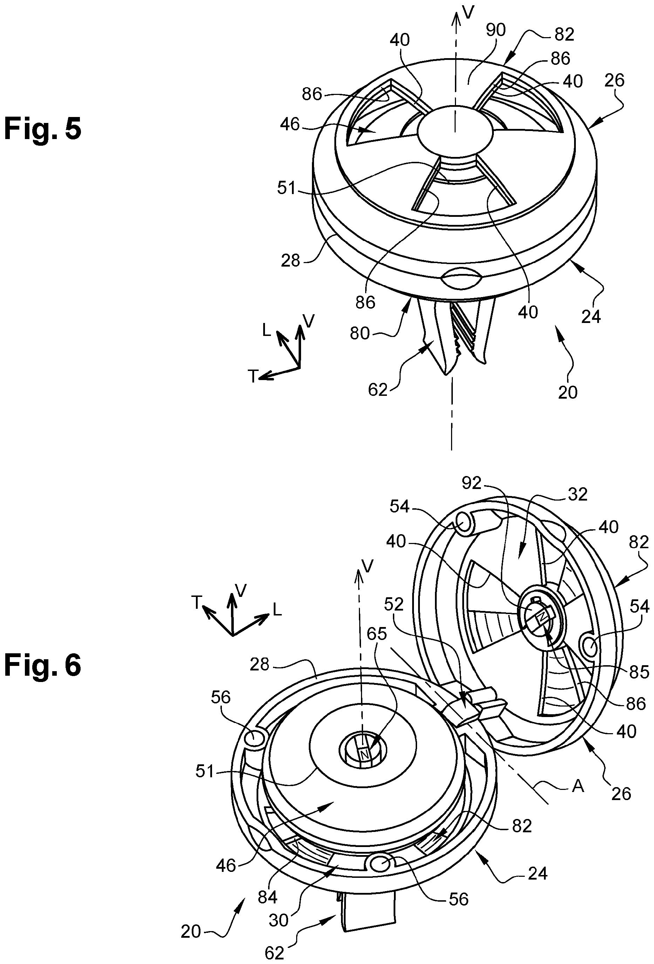

Comme on peut le voir notamment aux figures 6 et 7, la position ouverte du boîtier avant 22 et une position fonctionnelle dite de rechargement permettant la mise en place d'un support 46 dans le boîtier avant 22.

Le demi-boîtier avant 26 est maintenu en position fermée d'utilisation sur le demi-boîtier arrière 24 par des moyens magnétiques constitués ici de deux aimants 54 qui coopèrent avec des éléments magnétiques complémentaires 56 qui sont par exemple des aimants de polarité opposée.

Le diffuseur 20 comporte une tige arrière de fixation 60 qui s'étend selon l'axe V et dont la portion d'extrémité arrière est ici conformée en une pince élastique 62 qui, de manière connue, permet le montage et la fixation du diffuseur 20 par exemple sur un barreau d'une grille d'aération (Non représentée).

La tige arrière de fixation 60 se prolonge axialement vers l'avant par un arbre central 64 qui lui est assemblé et lié en rotation par une tige transversale d'assemblage 66.

L'arbre central 64 s'étend axialement à travers le trou central 34 du demi-boîtier arrière 24 et il comporte une collerette radiale 68 dont la face axiale arrière 70 est de profil convexe complémentaire du profil du premier siège central annulaire tronconique concave 36.

Au-delà de la collerette radiale 68, l'arbre central 64 se prolonge axialement vers l'avant à l'intérieur du boîtier avant 22 et à travers l'alésage central 47 du support 46, et l'arbre central 64 délimité axialement par une face orthogonale horizontale d'extrémité libre 72. En position fermée du boîtier avant 22, la face 72 d'extrémité libre de l'arbre central 64 s'étend sensiblement dans le plan du bord supérieur 51 qui délimite la surface concave 50 qui s'étend dans le plan de la face avant du support 46.

La face 72 d'extrémité libre de l'arbre central 64 est ainsi agencée à proximité de la portion centrale en vis-à-vis de la paroi latérale avant 32 du demi-boîtier avant 26 qui entoure le trou central 35.

Dans sa face 72 d'extrémité libre, l'arbre central 64 comporte ici un aimant arrière 65 de pôle magnétique opposé.

Pour l'occultation contrôlée des fenêtres arrière et avant du boîtier avant 22, le diffuseur 20 comporte un volet arrière d'obturation 80 et un volet avant d'obturation 82, aussi appelés volets d'occultation ou occulteurs.

Chaque volet d'obturation 80,82 est ici un volet extérieur au boîtier avant 22.

En position fermée d'utilisation, le boîtier 22 est monté tournant autour de l'axe V et est agencé entre le volet d'obturation arrière 80 et le volet d'obturation avant 82.

Chaque volet d'obturation 80,82 se présente sous la forme d'une plaque en forme de disque dont la forme et les dimensions sont complémentaires de celles de la paroi latérale, arrière 30 et avant 32 respectivement, à laquelle il est adjacent axialement.

Chaque volet d'obturation 80, 82 comporte ici trois ouvertures, arrière d'entrée 84 et avant de sortie 86 respectivement, de forme générale en secteur triangulaire qui sont réparties angulairement de manière régulière autour de l'axe V et qui sont séparées l'une de l'autre par trois portions pleines de forme générale en secteur triangulaire 88, 90.

Ainsi, chaque ouverture 84, 86 s'étend ici sensiblement sur cinquante-huit degrés d'angle.

La conception du diffuseur 20 est telle que, en position fermée du boîtier, les deux volets d'obturation arrière 80 et avant 82 sont solidaires angulairement l'un de l'autre et les ouvertures 84, 86 sont alignées angulairement les unes par rapport aux autres.

Les formes et dimensions des ouvertures 84 et 86 sont globalement identiques à celles des fenêtres associées 38 et 40 du boîtier avant 22 de telle manière que, en fonction de la position angulaire du boîtier avant 22 par rapport aux volets d'obturation 80 et 82, les fenêtres 38, 40 peuvent être entièrement ou partiellement occultées par les volets d'obturation 80, 82, ou bien entièrement dégagées pour un débit maximal de flux d'air parfumé.

La conception des ouvertures 84 et 86 n'est pas limitée à celle qui est représenté aux figures et elles peuvent revêtir toute(s) forme(s) adaptée(s).

La portion centrale 81 du volet arrière d'obturation 80 est liée en rotation à la tige axiale arrière de fixation 60 et à l'arbre central 64.

Le volet arrière d'obturation 80 s'étend vis-à-vis de la face externe 29 de la paroi latérale arrière 30 du demi-boîtier arrière 24 avec un léger jeu axial afin de ne pas frotter contre cette dernière 29 lors de la rotation du boîtier avant 22 par rapport aux volets d'obturation 80 et 82.

Le volet avant d'obturation 82 est monté libre en rotation sur le demi-boîtier avant 26.

À cet effet, la partie centrale 83 du volet avant d'obturation 82 est conformée en une tige qui s'étend axialement vers l'arrière à travers le trou central 35 de la paroi latérale avant 32 et elle est retenue axialement par rapport à cette dernière par l'intermédiaire d'un anneau élastique fendu 88.

La face d'extrémité libre arrière 92 de la tige centrale 83 s'étend dans un plan horizontal orthogonal à l'axe de libre rotation du volet avant d'obturation 82 par rapport au demi-boîtier avant 26.

Le volet avant d'obturation 82 s'étend vis-à-vis de la face externe 31 de la paroi latérale avant 32 du demi-boîtier arrière 24 avec un léger jeu axial afin de ne pas frotter contre cette dernière 31 lors de la rotation du boîtier avant 22 par rapport aux volets d'obturation 80 et 82.

Dans sa face 92 d'extrémité libre arrière, la tige centrale 83 comporte ici une paire avant 85 d'aimants adjacents de pôles magnétiques opposés Nord et Sud (N, S).

La longueur axiale de la tige centrale 83 est telle que, en position fermée du boîtier, et comme on peut le voir notamment aux figures 3 et 8, les deux faces d'extrémité libre 72 et 92 sont adjacentes, voire en contact.

En position fermée du boîtier avant 22, les deux paires d'aimants arrière 65 et avant 85 assurent une double fonction.

Premièrement, ces deux paires d'aimants 65 et 85 constituent un dispositif de liaison en rotation qui réalise un accouplement mécanique en rotation des deux volets d'obturation arrière 80 et avant 82.

Deuxièmement, ces deux paires d'aimants 65 et 85 réalisent un positionnement angulaire déterminé du volet d'obturation avant 82 par rapport aux volets d'obturation arrière 80 de telle manière que les ouvertures 84 et 86 soient alignées.

À cet effet, l'orientation angulaire de chaque paire d'aimants arrière 65, avant 85 par rapport au composant qui la porte est déterminée pour obtenir un tel positionnement angulaire relatif, comme illustré par exemple aux figures 3, 4 et 8.

A titre de variante, chaque paire d'aimants arrière 65 et avant 85 peut être remplacée par un seul aimant.

En position fermée d'utilisation du boîtier avant 22, et lorsque le diffuseur 20 est fixé par exemple une grille d'aération, il suffit de faire tourner le boîtier avant 22 autour de l'axe V, dans l'un ou l'autre des deux sens, pour faire varier sa position angulaire par rapport au couple de volets d'obturation 80 et 82 pour faire varier le débit du flux d'air traversant le boîtier avant 22 et/ou interrompre le fonctionnement du diffuseur 20.

La conception qui vient d'être décrite présente notamment les avantages suivants :
- lors de la fermeture du boîtier, il n'y a aucun frottement sur le support 60 ;
- on peut ouvrir le boitier avant 22 du diffuseur 20 quelle que soit la position angulaire relative de ce dernier par rapport aux volets d'obturation 80 et 82, sans que les deux volets d'obturation ne viennent buter l'un sur l'autre au niveau de la charnière 52 ;
- lors de la fermeture du boîtier 22, l'utilisateur n'a pas à se préoccuper de la position angulaire du volet d'obturation avant 82 par rapport au demi-boîtier avant 26

L'invention n'est pas limitée au mode de réalisation qui vient d'être décrit.

Notamment, l'assemblage et la fixation du demi-boîtier avant 26 sur le demi-boîtier arrière peuvent être différents.

Il est par exemple possible de prévoir un assemblage par emboîtement axial élastique avec des moyens de positionnement relatif angulaire des demi-boîtiers.

### Deuxième exemple de réalisation

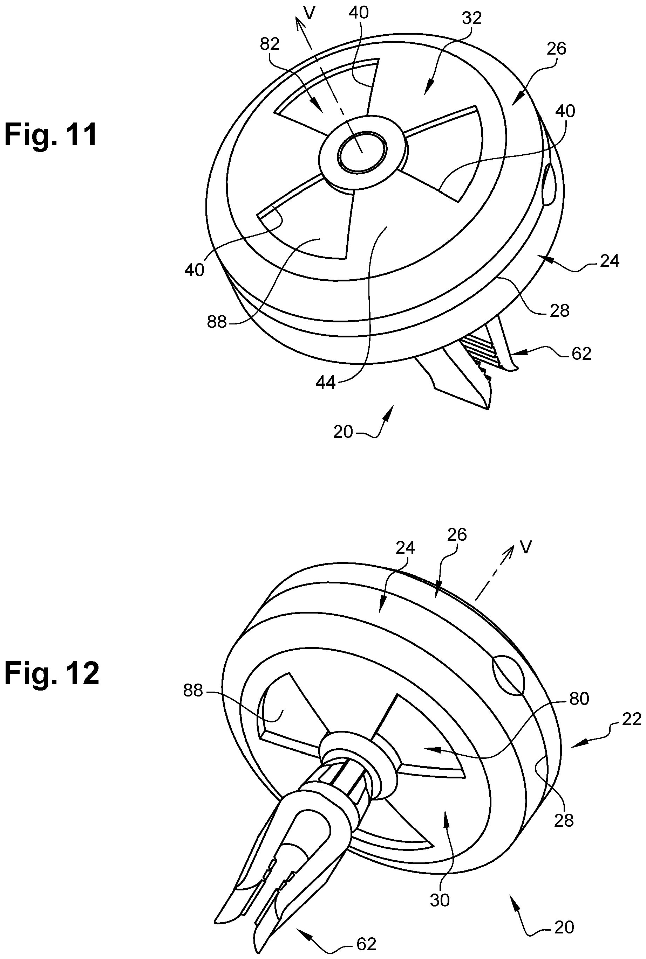

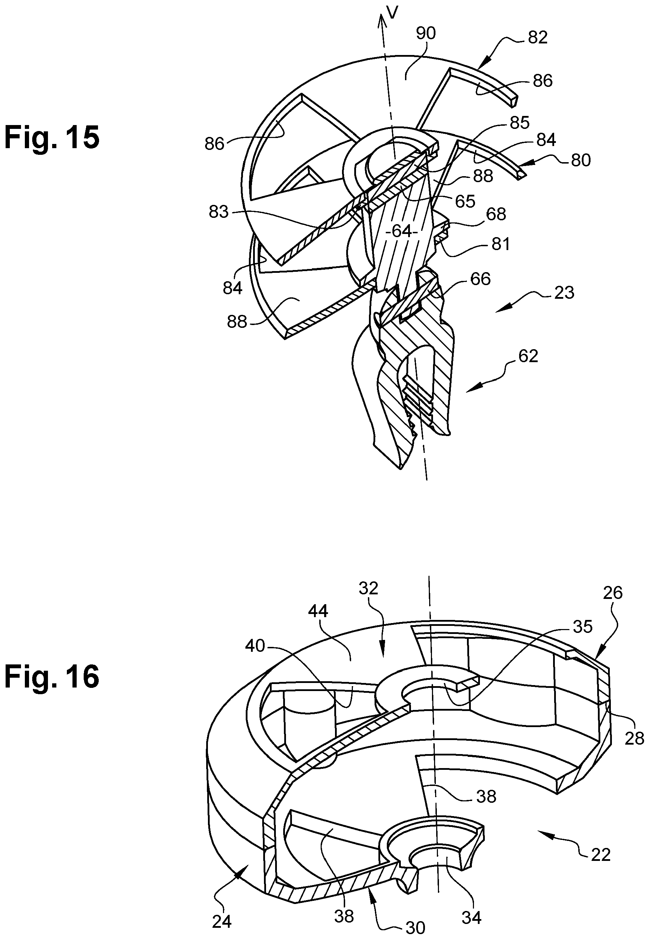

On a représenté aux figures 11 à 17, un deuxième exemple de réalisation dans lequel les deux volets d'obturation externes opposés arrière 80 et avant 82 d'obturation sont agencés tous les deux axialement à l'intérieur du boîtier avant 22.

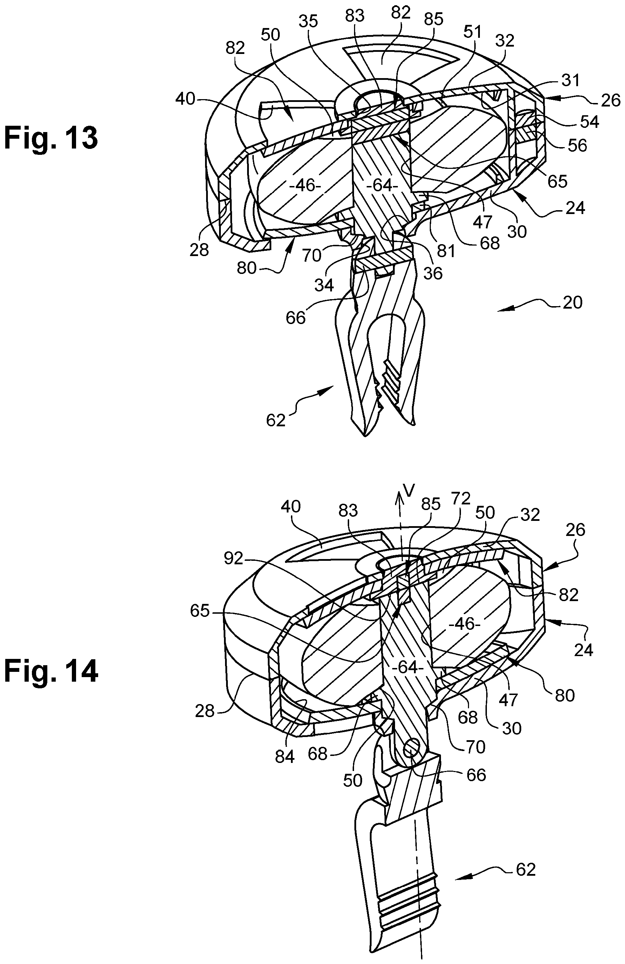

Ainsi, comme on peut le voir notamment aux figures 13 et 14, chaque volet d'obturation, arrière 80, avant 82, est adjacent - avec un léger jeu - à la face interne en vis-à-vis de la paroi associée, arrière 30 et avant 32 respectivement, du boîtier avant 22.

Une telle conception permet notamment une très grande variété de dessin et d'ornementation du boîtier avant 22, les surfaces externes du boîtier avant 22, et notamment celle de sa paroi avant 32, étant toujours entièrement visibles, quelle que soit la position angulaire du boîtier avant 22 par rapport à l'ensemble des deux volets d'obturation 80 et 82, et notamment lorsque les volets couplés d'obturation 80 et 82 sont en position fermée d'occultation complète des ouvertures du boîtier avant 22 (Voir figures 11 et 12).

Le support 46 est en appui axial sur la face supérieure annulaire convexe de la collerette 68 de l'arbre 64 qui constitue le siège complémentaire de la surface centrale annulaire concave 50 du support 46.

Le diamètre externe de l'arbre 64 est plus important et correspond au diamètre interne de l'alésage axial 47 du support 46.

On notera que l'arbre central 64 sur lequel repose le support 46 est fixe lors de la rotation du boîtier 22 et que, en conséquence, il n'y a pas de frottement d'usure sur le support 46 lors de la rotation du boîtier 22.

### Troisième exemple de réalisation

Dans un troisième exemple, non représenté aux figures, il est possible prévoir que seulement un seul des deux volets externes opposés d'obturation est agencé axialement à l'intérieur du boîtier avant, et par exemple le volet avant 82 adjacent à la paroi avant 32 du boîtier avant 22.

## Revendications

1. Diffuseur (20) de parfum qui comporte :
- un élément arrière (62) de fixation du diffuseur (20) ;
- un boîtier avant (22), dans lequel deux parois axialement opposées arrière (30) et avant (32) du boîtier avant (22) comportent chacune au moins une fenêtre, arrière d'entrée (38) et avant de sortie (40) respectivement, pour permettre la formation d'un flux d'air s'écoulant axialement à travers le boîtier avant (22) et la diffusion d'un flux d'air parfumé hors du boîtier avant (22) ;
- un support (46) d'un matériau parfumé logé dans le boîtier avant (22) ;
- deux volets externes opposés, arrière (80) et avant (82), d'obturation dont chacun est adjacent à une paroi associée, arrière (30) et avant (32) respectivement, du boîtier avant (22), qui sont accouplés pour former un ensemble de volets d'obturation (80, 82) qui est monté tournant par rapport au boîtier avant (22), et dont chacun comporte au moins une ouverture, arrière d'entrée (84) et avant (86) de sortie respectivement, pour permettre le passage du flux d'air à travers le boîtier avant (22) lorsque ces ouvertures (84, 86) sont positionnées angulairement au moins partiellement en vis-à-vis des fenêtres arrière (38) et avant (40) associées du boîtier avant (22) ; dans lequel l'ensemble de volets d'obturation (80, 82) est lié en rotation à l'élément arrière de fixation (62) et le boîtier avant (22) est monté tournant par rapport à l'élément arrière (62) de fixation pour permettre un réglage de la position angulaire du boîtier avant (22) par rapport à l'ensemble de volets d'obturation (80, 82).

2. Diffuseur (20) selon la revendication 1, **caractérisé en ce que** l'élément arrière de fixation (62) comporte une tige axiale (60) de fixation qui est liée en rotation au volet arrière d'obturation (80) et se prolonge vers l'avant à l'intérieur du boîtier avant (22).

3. Diffuseur (20) selon la revendication 2, **caractérisé en ce que** le boîtier avant (22) est un boîtier rechargeable comportant :
- un demi-boîtier arrière (24) qui est monté tournant par rapport à l'arbre central (64) ;
- un demi-boîtier avant (26) qui est porté par le demi-boîtier arrière (24) sur lequel il est monté mobile entre une position fermée d'utilisation et une position ouverte permettant le rechargement du boîtier avant (22), dans lequel le volet avant d'obturation (82) est monté libre en rotation par rapport au demi-boîtier avant (26),et **en ce que** le diffuseur (20) comporte un dispositif de liaison en rotation du volet avant d'obturation (82) avec l'arbre central (64) lorsque le demi-boîtier avant (26) est en position fermée.

4. Dispositif selon la revendication 3, **caractérisé en ce que** le demi-boîtier avant (26) est monté pivotant sur le demi-boîtier arrière (24), entre ses positions fermée d'utilisation et ouverte de rechargement, autour d'un axe (A) de pivotement orthogonal à l'axe de rotation du boîtier avant (22).

5. Diffuseur (20) selon l'une des revendications 3 ou 4, **caractérisé en ce qu'**il comporte des moyens magnétiques (54, 56), agencés entre les deux demi-boîtiers arrière (24) et avant (26), qui maintiennent le demi-boîtier avant (26) en position fermée d'utilisation.

6. Diffuseur (20) selon l'une quelconque des revendications 3 à 5, **caractérisé en ce que** le dispositif de liaison en rotation du volet avant d'obturation (82) avec l'arbre central (64) est un système magnétique comportant au moins un aimant.

7. Diffuseur (20) selon la revendication 6, **caractérisé en ce que** le dispositif de liaison en rotation du volet avant d'obturation (82) avec l'arbre central (64) est un système magnétique de positionnement angulaire automatique, à la fermeture du boîtier avant (22), du volet avant d'obturation (82) par rapport à l'arbre central (64), et ainsi par rapport au volet arrière d'obturation (80).

8. Diffuseur (20) selon la revendication 7, **caractérisé en ce que** le système magnétique de positionnement angulaire automatique, à la fermeture du boîtier avant (22), du volet avant d'obturation (82) par rapport à l'arbre central (64) comporte :
- un élément magnétique arrière (65) à aimantation permanente agencé à l'extrémité avant de l'arbre central (64) qui comprend au moins deux pôles magnétiques opposés ;
- un élément magnétique (85) avant à aimantation permanente porté par le volet avant d'obturation (82) et agencé en vis-à-vis du premier élément magnétique (65) qui comprend deux pôles magnétiques opposés complémentaires des deux pôles magnétiques opposés du premier élément magnétique (65).

9. Diffuseur (20) selon la revendication 7, **caractérisé en ce que** le support (46) d'un matériau parfumé comporte une face avant (61) orthogonale à l'axe de l'arbre central (64), et **en ce que** ladite extrémité avant (72) de l'arbre central (64) s'étend sensiblement dans le plan de ladite face avant.

10. Diffuseur (20) selon la revendication 3, **caractérisé en ce que** le demi-boîtier avant (26) est emboîté élastiquement sur le demi-boîtier arrière (24).

11. Diffuseur (20) selon l'une quelconque des revendications 3 à 10, **caractérisé en ce que** le boîtier avant (26) est partagé en deux demi-boîtiers arrière et avant selon un plan de joint (28) orthogonal monté vissé sur le demi-boîtier arrière (24).

12. Diffuseur selon l'une quelconque des revendications précédentes, **caractérisé en ce que** le boîtier avant (22) est agencé axialement entre les deux volets externes opposés arrière (80) et avant (82) d'obturation.

13. Diffuseur selon l'une quelconque des revendications 1 à 11, **caractérisé en ce que** les deux volets externes opposés arrière (80) et avant (82) d'obturation sont agencés axialement à l'intérieur du boîtier avant (22), chacun adjacent à la paroi associée, arrière (30) et avant (32) respectivement, du boîtier avant (22).

14. Diffuseur selon l'une quelconque des revendications 1 à 11, **caractérisé en ce qu'**un seulement des deux volets externes opposés arrière (80) ou avant (82) d'obturation est agencé axialement à l'intérieur du boîtier avant (22) adjacent à la paroi associée, arrière (30) ou avant (32) respectivement, du boîtier avant (22).

## Patentansprüche

1. Duftspender (20), der aufweist:
- ein hinteres Element (62) zum Befestigen des Spenders (20);
- ein vorderes Gehäuse (22), in dem zwei axial gegenüberliegende Wände, eine hintere (30) und eine vordere (32), des vorderen Gehäuses (22) jeweils mindestens ein Fenster, einen hinteren Einlass (38) beziehungsweise einen vorderen Auslass (40) zum Ermöglichen des Bildens eines Luftstroms umfassen, der axial durch das vordere Gehäuse (22) strömt, und des Spendens eines Stroms von parfümierter Luft aus dem vorderen Gehäuse (22);
- einen Träger (46) eines parfümierten Materials, der in dem vorderen Gehäuse (22) untergebracht ist;
- zwei gegenüberliegende Außenverschlussklappen, eine hintere (80) und eine vordere (82), von denen jede an eine zugehörige Wand, eine hintere (30) beziehungsweise vordere (32), des vorderen Gehäuses (22) angrenzt, die zum Bilden einer Anordnung von Verschlussklappen (80, 82) gekoppelt sind, die relativ zu dem vorderen Gehäuse (22) drehbar montiert ist und von denen jede mindestens eine Öffnung, einen hinteren Einlass (84) beziehungsweise einen vorderen Auslass (86), aufweist, um den Durchgang des Luftstroms durch das vordere Gehäuse (22) zu ermöglichen, wenn diese Öffnungen (84, 86) mindestens teilweise gegenüber dem zugehörigen hinteren (38) und vorderen (40) Fenster des vorderen Gehäuses (22) winkelmäßig positioniert sind; wobei die Anordnung von Verschlussklappen (80, 82) mit dem hinteren Befestigungselement (62) drehbar verbunden ist und das vordere Gehäuse (22) relativ zu dem hinteren Befestigungselement (62) drehbar montiert ist, um eine Regelung der Winkelposition des vorderen Gehäuses (22) relativ zu der Anordnung von Verschlussklappen (80, 82) zu ermöglichen.

2. Spender (20) nach Anspruch 1, **dadurch gekennzeichnet, dass** das hintere Befestigungselement (62) eine axiale Befestigungsstange (60) aufweist, die mit der hinteren Verschlussklappe (80) drehbar verbunden ist und sich nach vorne innerhalb des vorderen Gehäuses (22) erstreckt.

3. Spender (20) nach Anspruch 2, **dadurch gekennzeichnet, dass** das vordere Gehäuse (22) ein nachfüllbares Gehäuse ist, aufweisend:
- eine hintere Gehäusehälfte (24), die relativ zu der zentralen Welle (64) drehbar montiert ist;
- eine vordere Gehäusehälfte (26), die durch die hintere Gehäusehälfte (24) getragen wird, auf der sie zwischen einer geschlossenen Verwendungsposition und einer offenen Position, die das Nachfüllen des vorderen Gehäuses (22) ermöglicht, bewegbar montiert ist, wobei die vordere Verschlussklappe (82) relativ zu der vorderen Gehäusehälfte (26) frei drehend montiert ist, und dass der Spender (20) eine Vorrichtung für eine Drehverbindung der vorderen Verschlussklappe (82) mit der zentralen Welle (64) aufweist, wenn die vordere Gehäusehälfte (26) sich in der geschlossenen Position befindet.

4. Vorrichtung nach Anspruch 3, **dadurch gekennzeichnet, dass** die vordere Gehäusehälfte (26) zwischen ihrer geschlossenen Verwendungsposition und offenen Nachfüllposition um eine Schwenkachse (A) orthogonal zu der Drehachse des vorderen Gehäuses (22) an der hinteren Gehäusehälfte (24) schwenkbar montiert ist.

5. Spender (20) nach einem der Ansprüche 3 oder 4,
**dadurch gekennzeichnet, dass** er Magnetmittel (54, 56) aufweist, die zwischen den zwei Gehäusehälften, der hinteren (24) und der vorderen (26), angeordnet sind, die die vordere Gehäusehälfte (26) in der geschlossenen Verwendungsposition halten.

6. Spender (20) nach einem der Ansprüche 3 bis 5,
**dadurch gekennzeichnet, dass** die Vorrichtung für die Drehverbindung der vorderen Verschlussklappe (82) mit der zentralen Welle (64) ein Magnetsystem, aufweisend mindestens einen Magneten, ist.

7. Spender (20) nach Anspruch 6, **dadurch gekennzeichnet, dass** die Vorrichtung für die Drehverbindung der vorderen Verschlussklappe (82) mit der zentralen Welle (64) ein Magnetsystem für eine automatische Winkelpositionierung der vorderen Verschlussklappe (82) relativ zu der zentralen Welle (64), bei dem Schließen des vorderen Gehäuses (22), und auch relativ zu der hinteren Verschlussklappe (80) ist.

8. Spender (20) nach Anspruch 7, **dadurch gekennzeichnet, dass** das Magnetsystem für die automatische Winkelpositionierung, bei dem Schließen des vorderen Gehäuses (22), der vorderen Verschlussklappe (82) relativ zu der zentralen Welle (64) aufweist:
- ein dauermagnetisches hinteres Magnetelement (65), das an dem vorderen Ende der zentralen Welle (64) angeordnet ist, das mindestens zwei gegenüberliegende Magnetpole umfasst;
- ein dauermagnetisches vorderes Magnetelement (85), das durch die vordere Verschlussklappe (82) getragen wird und gegenüber dem ersten Magnetelement (65) angeordnet ist, das zwei gegenüberliegende Magnetpole umfasst, die zu den zwei gegenüberliegenden Magnetpolen des ersten Magnetelements (65) komplementär sind.

9. Spender (20) nach Anspruch 7, **dadurch gekennzeichnet, dass** der Träger (46) eines parfümierten Materials eine Vorderseite (61) aufweist, die orthogonal zu der Achse der zentralen Welle (64) ist, und **dass** das vordere Ende (72) der zentralen Welle (64) sich im Wesentlichen in der Ebene der Vorderseite erstreckt.

10. Spender (20) nach Anspruch 3, **dadurch gekennzeichnet, dass** die vordere Gehäusehälfte (26) an der hinteren Gehäusehälfte (24) elastisch eingefügt ist.

11. Spender (20) nach einem der Ansprüche 3 bis 10,
**dadurch gekennzeichnet, dass** das vordere Gehäuse (26) entlang einer orthogonalen Verbindungsebene (28) in zwei Gehäusehälften, eine hintere und eine vordere, geteilt ist, die mit der hinteren Gehäusehälfte (24) verschraubt ist.

12. Spender nach einem der vorstehenden Ansprüche,
**dadurch gekennzeichnet, dass** das vordere Gehäuse (22) zwischen den zwei gegenüberliegenden äußeren Verschlussklappen, der hinteren (80) und der vorderen (82), angeordnet ist.

13. Spender nach einem der Ansprüche 1 bis 11,
**dadurch gekennzeichnet, dass** die zwei gegenüberliegenden äußeren Verschlussklappen, die hintere (80) und die vordere (82), innerhalb des vorderen Gehäuses (22) jeweils angrenzend an die zugehörige Wand, die hintere (30) beziehungsweise die vordere (32), des vorderen Gehäuses (22) axial angeordnet sind.

14. Spender nach einem der Ansprüche 1 bis 11,
**dadurch gekennzeichnet, dass** lediglich eine der zwei gegenüberliegenden äußeren Verschlussklappen, der hinteren (80) oder der vorderen (82), innerhalb des vorderen Gehäuses (22) angrenzend an die zugehörige Wand, die hintere (30) beziehungsweise die vordere (32), des vorderen Gehäuses (22) angeordnet ist.

## Claims

1. A fragrance dispenser (20) comprising:
- a rear element (62) for fastening the diffuser (20);
- a front casing (22), wherein two axially opposed rear (30) and front (32) walls of the front casing (22) each comprise at least one window, respectively a rear inlet window (38) and front outlet window (40), to allow the formation of an airflow circulating axially through the front casing (22) and the diffusion of a scented air flow out of the front casing (22);
- a scented material holder (46) housed in the front casing (22);
- two opposing external shutters, rear (80) and front (82), each of which is adjacent to an associated wall, rear (30) and front (32) respectively, of the front casing (22), which are coupled to form a shutter assembly (80, 82) which is rotatably mounted relative to the front casing (22), and each of which has at least one aperture, respectively a rear inlet aperture (84) and a front outlet aperture (86), to allow airflow through the front casing (22) when these apertures (84, 86) are angularly positioned at least partially opposite the associated rear (38) and front (40) windows of the front casing (22); wherein the shutter assembly (80, 82) is rotatably connected to the rear fastening element (62) and the front casing (22) is rotatably mounted relative to the rear fastening element (62) to allow adjustment of the angular position of the front casing (22) relative to the shutter assembly (80, 82).

2. The diffuser (20) according to claim 1, **characterized in that** the rear fastening element (62) comprises an axial fastening rod (60) which is rotationally connected to the rear shutter (80) and extends forward into the front casing (22).

3. The diffuser (20) according to claim 2, **characterized in that** the front casing (22) is a reloadable casing comprising:
- a rear half-casing (24) rotatably mounted relative to the central shaft (64);
- a front half-casing (26) which is carried by the rear half-casing (24) on which it is mounted so as to be movable between a closed position for use and an open position for reloading the front casing (22), wherein the front shutter (82) is mounted so as to rotate freely relative to the front half-casing (26),and **in that** the diffuser (20) comprises a device for rotationally connecting the front shutter (82) to the central shaft (64) when the front half-casing (26) is in the closed position.

4. The device according to claim 3, **characterized in that** the front half-casing (26) is pivotably mounted on the rear half-casing (24), between its closed position for use and its open position for reloading, about a pivot axis (A) orthogonal to the axis of rotation of the front casing (22).

5. The diffuser (20) according to one of claims 3 or 4, **characterized in that** it comprises magnetic means (54, 56), arranged between the two rear (24) and front (26) half-casings, which hold the front half-casing (26) in the closed position of use.

6. The diffuser (20) according to any one of claims 3 to 5,
**characterized in that** the device for rotationally connecting the front shutter (82) to the central shaft (64) is a magnetic system comprising at least one magnet.

7. The diffuser (20) according to claim 6, **characterized in that** the device for rotationally connecting the front shutter (82) to the central shaft (64) is a magnetic system for automatic angular positioning of the front shutter (82) relative to the central shaft (64), and thus relative to the rear shutter (80), when the front casing (22) is closed.

8. The diffuser (20) according to claim 7, **characterized in that** the magnetic system for automatic angular positioning of the front shutter (82) relative to the central shaft (64), when the front casing (22) is closed, comprises:
- a permanently magnetized rear magnetic element (65) arranged at the front end of the central shaft (64) and comprising at least two opposite magnetic poles;
- a permanently magnetized front magnetic element (85) carried by the front shutter (82) and arranged opposite the first magnetic element (65), which comprises two opposite magnetic poles complementary to the two opposite magnetic poles of the first magnetic element (65).

9. The diffuser (20) according to claim 7, **characterized in that** the scented material holder (46) for a has a front face (61) orthogonal to the axis of the central shaft (64), and **in that** said front end (72) of the central shaft (64) extends substantially in the plane of said front face.

10. The diffuser (20) according to claim 3, **characterized in that** the front half-casing (26) is elastically fitted onto the rear half-casing (24).

11. The diffuser (20) according to any one of claims 3 to 10,
**characterized in that** the front casing (26) is divided into two rear and front half-casings along an orthogonal parting line (28) screw-mounted on the rear half-casing (24).

12. The diffuser according to any one of the preceding claims,
**characterized in that** the front casing (22) is arranged axially between the two opposing external rear (80) and front (82) shutters.

13. The diffuser according to any one of claims 1 to 11,
**characterized in that** the two opposing external rear (80) and front (82) shutters are arranged axially inside the front casing (22), each adjacent to the associated rear (30) and front (32) wall, respectively, of the front casing (22).

14. The diffuser according to any one of claims 1 to 11,
**characterized in that** only one of the two opposing external rear (80) or front (82) shutters is arranged axially inside the front casing (22) adjacent to the associated rear (30) or front (32) wall, respectively, of the front casing (22).
